(19) Europäisches Patentamt
European Patent Office
Office européen des brevets

(11) Numéro de publication : **0 208 622**
**B1**

(12) # FASCICULE DE BREVET EUROPÉEN

(45) Date de publication du fascicule du brevet : **19.07.89**

(51) Int. Cl.[4] : **C 07 D457/08**, C 07 D457/04

(21) Numéro de dépôt : **86401538.3**

(22) Date de dépôt : **10.07.86**

(54) **Procédé de préparation de dérivés N-méthyles de l'ergoline.**

(30) Priorité : **11.07.85 FR 8510620**

(43) Date de publication de la demande :
**14.01.87 Bulletin 87/03**

(45) Mention de la délivrance du brevet :
**19.07.89 Bulletin 89/29**

(84) Etats contractants désignés :
**AT BE CH DE FR GB IT LI LU NL SE**

(56) Documents cités :
**EP-A- 0 000 533**
**DE-A- 2 618 033**
**DE-A- 3 007 196**
**Le dossier contient des informations techniques présentées postérieurement au dépôt de la demande et ne figurant pas dans le présent fascicule.**

(73) Titulaire : **RHONE-POULENC SANTE**
**20, avenue Raymond Aron**
**F-92160 Antony (FR)**

(72) Inventeur : **Gervais, Christian**
**6 allée Marcel Achard**
**F-69100 Villeurbanne (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

## Description

La présente invention concerne un procédé de préparation de dérivés N-méthylés de l'ergoline de formule générale :

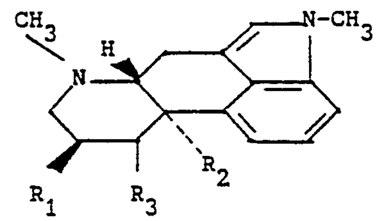

(I)

dans laquelle $R_1$ représentant un radical carboxy ou alcoyloxycarbonyle dont la partie alcoyle contient 1 à 4 atomes de carbone, $R_2$ représente un atome d'hydrogène ou un radical méthoxy, $R_3$ représente un atome d'hydrogène ou bien forme avec $R_2$ une liaison et $R_1$ représentant un radical hydroxyméthyle, $R_2$ représente un atome d'hydrogène ou un radical méthoxy et $R_3$ représente un atome d'hydrogène.

Les produits de formule générale (I) sont des intermédiaires particulièrement intéressants pour préparer des composés à activité pharmacologique tels que des vasodilatateurs périphériques et cérébraux (nicergoline) ou des antimigraineux (méthysergide, métergoline).

Il est connu, de manière générale, de réaliser la méthylation d'un noyau indolique au moyen d'un agent de méthylation choisi parmi les halogénures de méthyle ou le sulfate de diméthyle en présence d'une base choisie parmi les hydroxydes, hydrures, amidures ou alcoolates de métaux alcalins ou les dérivés organo-métalliques tels que les dérivés organo-magnésiens ou organo-lithiens [cf. M. G. Reinecke, J. Org. Chem., 37 (20) 3066 (1972)]. Cependant cette technique ne donne pas des résultats satisfaisants lorsque le noyau indolique porte des substituants azotés ou hydroxylés.

Dans le brevet français 1 359 563 est décrite la méthylation de l'atome d'azote du noyau ergoline par action d'un halogénure de méthyle dans l'ammoniac liquide en présence de potassium métallique.

Dans les demandes de brevets allemands DE-2 618 033 et DE-3 007 196 est décrite la méthylation d'une aniline au moyen de carbonate de méthyle à une température comprise entre 180 et 250 °C ou au moyen de carbonate de méthyle et d'iodure de méthyle à une température voisine de 150 °C.

Dans la demande de brevet français FR-2 478 639 est décrite la méthylation d'un dérivé particulier de l'indole, l'[(indolyl-3)-2 éthyl]-1 pyrrolidone-2, au moyen de carbonate de méthyle en présence d'hydrure de sodium par chauffage pendant 6 heures à la température de reflux du tétrahydrofuranne. Cependant l'application de ce procédé à la méthylation de dérivés de l'ergoline ne conduit pas à des résultats satisfaisants.

Dans la demande de brevet européen EP-A-0 000 533 est décrite la méthylation du lumilysergate de méthyle au moyen d'agents habituels de méthylation tels que les halogénures de méthyle ou le sulfate de méthyle en présence d'une solution aqueuse de soude à 45-50% utilisée en catalyse de transfert de phase liquide-liquide.

Il a maintenant été trouvé, et c'est ce qui fait l'objet de la présente invention, que les dérivés de l'ergoline de formule générale (I) peuvent être obtenus par méthylation d'un dérivé de l'ergoline de formule générale :

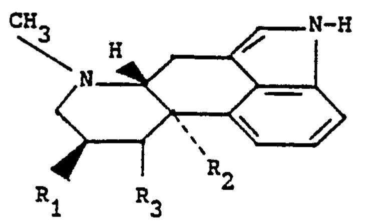

(II)

dans laquelle $R_1$, $R_2$ et $R_3$ sont définis comme précédemment, au moyen de carbonate de méthyle en présence d'un agent basique choisi parmi les hydrures ou alcoolates de métaux alcalins tels que les hydrures ou alcoolates de sodium ou de potassium en opérant dans un solvant aprotique apolaire en présence d'un catalyseur de transfert de phase choisi parmi les sels quaternaires tels que le chlorure de tétrabutylammonium.

La température de mise en œuvre du procédé est généralement comprise entre 0 et 100 °C et, de préférence, entre 40 et 60 °C

Comme solvants aprotiques apolaires peuvent être utilisés les éthers tels que le tétrahydrofuranne.

Par ailleurs, le procédé peut être mis en œuvre dans le carbonate de méthyle lui-même qui joue le rôle de solvant et qui, compte tenu de son point d'ébullition relativement bas (90 °C), peut être facilement éliminé.

Le procédé selon l'invention permet de réaliser la réaction de méthylation dans des conditions douces en utilisant des produits plus facilement manipulables que les autres agents de méthylation (halogénures de méthyle, sulfate de diméthyle), le carbonate de méthyle étant, en particulier, beaucoup moins toxique.

Lorsque le noyau ergoline est substitué par des fonctions amine tertiaire ou alcool primaire, il ne se produit pas, comme avec les autres agents de méthylation connus, de réactions secondaires de quaternisation de l'amine tertiaire ou d'éthérification de l'alcool primaire. Cependant dans ce dernier cas il peut être avantageux de traiter le produit de la réaction par une base telle que la soude avant d'isoler le produit final.

Lorsque le noyau ergoline est substitué par une fonction ester, en particulier une fonction ester méthylique, la fonction ester se trouve être préservée de réaction de saponification dans les conditions de mise en œuvre du procédé selon l'invention.

Les exemples suivants, donnés à titre non limitatif, montrent comment l'invention peut être mise en pratique.

## Exemple 1

Une solution de 314 mg de méthoxy-10α lumilysergate de méthyle ($10^{-3}$ mole) dans 25 $cm^3$ de carbonate de méthyle est versée dans un réacteur de $16.10^{-3}$ mole de méthylate de sodium et $1{,}75.10^{-3}$ mole de chlorure de tétrabutylammonium.

Le mélange réactionnel, maintenu à l'abri de l'humidité, est agité pendant 24 heures à une température voisine de 20 °C.

Le dosage du mélange réactionnel par chromatographie liquide à haute performance montre qu'il contient $0{,}8.10^{-3}$ mole de N-méthyl méthoxy-10α lumilysergate de méthyle.

Le taux de transformation est de 97%.

## Exemple 2

A une solution de 943 mg de méthoxy-10α lumilysergate de méthyle (3 m.moles) dans 100 $cm^3$ de carbonate de méthyle sont ajoutés successivement 1,5 m.mole de chlorure de tétrabutylammonium et 24 m.moles de méthylate de sodium.

Le mélange réactionnel, maintenu à l'abri de l'humidité, est agité pendant 3 heures 30 minutes à 60 °C. Le mélange réactionnel contient alors 2,83 m.moles de N-méthyl méthoxy-10α lumilysergate de méthyle, ce qui correspond à un rendement de 94 %.

Le taux de transformation du méthoxy-10α lumilysergate de méthyle est de 98,5 %.

On ajoute au mélange réactionnel 24 m.moles d'acide acétique puis filtre sur silice. On isole ainsi le N-méthyl méthoxy-10α lumilysergate de méthyle dont les caractéristiques sont confirmées par le spectre de résonance magnétique nucléaire du proton et par chromatographie liquide à haute performance.

## Exemple 3

On met en suspension 268 mg de méthoxy-10α lumilysergol (1 m.mole) dans 30 $cm^3$ d'un mélange de carbonate de méthyle et de tétrahydrofuranne (1-1 en volumes). On ajoute alors 8 m.moles de méthylate de sodium et 2 m.moles de chlorure de tétrabutylammonium. Le mélange réactionnel est agité pendant 5 heures à 60 °C. Les solvants sont évaporés sous pression réduite puis on ajoute 30 $cm^3$ d'un mélange méthanol-eau (4-1 en volumes). La solution hydrométhanolique est chauffée à 60 °C pendant 3 heures.

Le N-méthyl méthoxy-10α lumilysergol est isolé après précipitation par addition d'eau à une température voisine de 10 °C.

Le rendement en N-méthyl méthoxy-10α lumilysergol est de 96 %.

Le taux de conversion du méthoxy-10α lumilysergol est de 98 %.

La structure du produit obtenu est confirmée par le spectre de résonance magnétique nucléaire et par chromatographie liquide à haute performance.

## Exemple 4

On opère dans les conditions décrites dans l'exemple 2 mais en utilisant :

| | |
|---|---|
| — méthoxy-10α lumilysergate de méthyle | 1 m.mole |
| — carbonate de méthyle | 8 $cm^3$ |
| — chlorure de tétrabutylammonium | 0,02 m.mole |
| — méthylate de sodium | 8 m.moles |

Après 22 heures de chauffage à 60 °C, il se forme 0,72 m.mole de N-méthyl méthoxy-10α lumilysergate de méthyle avec un rendement de 72 %.

## Revendications

1. Procédé de préparation de dérivés N-méthylés de l'ergoline de formule générale :

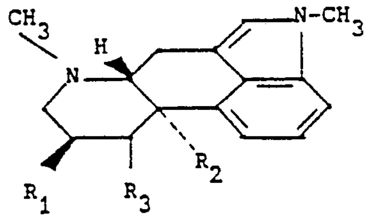

(I)

dans laquelle $R_1$ représentant un radical carboxy ou alcoyloxycarbonyle dont la partie alcoyle contient 1 à 4 atomes de carbone, $R_2$ représente un atome d'hydrogène ou un radical méthoxy et $R_3$ représente un atome d'hydrogène ou bien forme avec $R_2$ une liaison et $R_1$ représentant un radical hydroxyméthyle, $R_2$ représente un atome d'hydrogène ou un radical méthoxy et $R_3$ représente un atome d'hydrogène, par action du carbonate de méthyle en présence d'un agent basique sur un produit de formule générale :

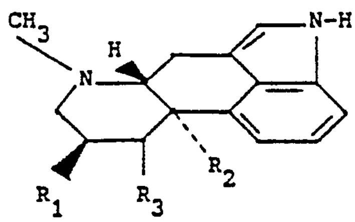

(II)

dans laquelle $R_1$, $R_2$ et $R_3$ sont définis comme précédemment, caractérisé en ce que l'on opère dans un solvant aprotique apolaire en présence d'un catalyseur de transfert de phase.

2. Procédé selon la revendication 1 caractérisé en ce que le solvant est le carbonate de méthyle.

3. Procédé selon la revendication 1 caractérisé en ce que le solvant est choisi parmi les éthers tels que le tétrahydrofuranne.

4. Procédé selon la revendication 1 caractérisé en ce que le catalyseur de transfert de phase est le chlorure de tétrabutylammonium.

5. Procédé selon l'une des revendications 1 à 4 caractérisé en ce que la température est comprise entre 0 et 100 °C.

## Claims

1. A process for preparing N-methyl derivatives of ergoline, of general formula :

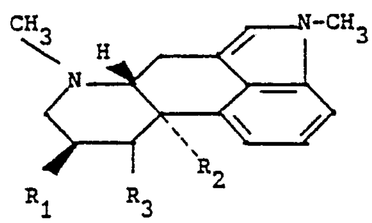

(I)

in which, when $R_1$ denotes a carboxy or alkoxycarbonyl radical in which the alkyl portion contains 1 to 4 carbon atoms, $R_2$ denotes a hydrogen atom or a methoxy radical and $R_3$ denotes a hydrogen atom or alternatively forms a bond with $R_2$, and when $R_1$ denotes a hydroxymethyl radical, $R_2$ denotes a hydrogen atom or a methoxy radical and $R_3$ denotes a hydrogen atom, by the action of methyl carbonate in the presence of a basic agent on a product of general formula :

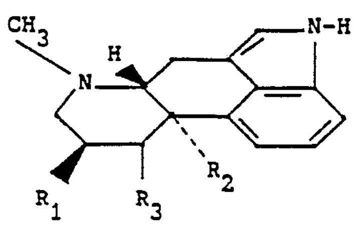

(II)

in which $R_1$, $R_2$ and $R_3$ are defined as above, wherein the procedure is carried out in an apolar aprotic solvent in the presence of a phase transfer catalyst.

2. A process according to claim 1, wherein the solvent is methyl carbonate.

3. A process according to claim 1, wherein the solvent is chosen from ethers such as tetrahydrofuran.

4. A process according to claim 1, wherein the phase transfer catalyst is tetrabutylammonium chloride.

5. A process according to one of claims 1 to 4, wherein the temperature is between 0 and 100 °C.

## Patentansprüche

1. Verfahren zur Herstellung von N-Methylverbindungen von Ergolin der allgemeinen Formel :

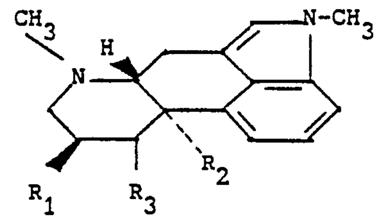

(I)

in welcher $R_1$ einen Carboxy- oder Alkyloxycarbonylrest darstellt, worin der Alkylteil 1 bis 4 Kohlenstoffatome enthält, $R_2$ ein Wasserstoffatom oder einen Methoxyrest darstellt und $R_3$ ein Wasserstoffatom darstellt oder zusammen mit $R_2$ eine Bindung darstellt und $R_1$ einen Hydroxymethylrest darstellt, $R_2$ ein Wasserstoffatom oder einen Methoxyrest darstellt und $R_3$ ein Wasserstoffatom darstellt, durch Einwirkung von Methylcarbonat auf eine Verbindung der allgemeinen Formel :

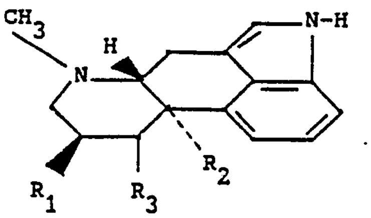

(II)

in welcher $R_1$, $R_2$ und $R_3$ die obige Bedeutung haben, in Gegenwart eines basischen Mittels, dadurch gekennzeichnet, daß man in einem apolaren aprotischen Lösungsmittel in Gegenwart eines Phasenübertragungskatalysators arbeitet.

2. Verfahren nach Anspruch 1, dadurch gekennzeichnet, daß das Lösungsmittel Methylcarbonat ist.

3. Verfahren nach Anspruch 1, dadurch gekennzeichnet, daß das Lösungsmittel aus den Äthern, z. B. Tetrahydrofuran, ausgewählt ist.

4. Verfahren nach Anspruch 1, dadurch gekennzeichnet, daß der Phasenübertragungskatalysator Tetrabutylammoniumchlorid ist.

5. Verfahren nach einem der Ansprüche 1 bis 4, dadurch gekennzeichnet, daß die Temperatur zwischen 0 und 100 °C liegt.